# EUROPEAN PATENT APPLICATION

(11) **EP 4 847 605 A2**
(43) Date of publication of application: **02.12.2026**
(21) Application number: 26209519.3
(22) Date of filing: 21.03.2023
(51) Int. Cl.: C12N 5/077

(54) **TREATMENT OF ERECTILE DYSFUNCTION USING MESENCHYMAL STEM CELLS OF AMNIOTIC FLUID**

(30) Priority: 21.03.2022 US 202263321887 P
(62) Document number(s) of the earlier application(s) in accordance with Art. 76 EPC: 23773862.0 / 4 479 069
(71) Applicant: U-Neuron Biomedical Inc., New Taipei City 22175 (TW)
(72) Inventor: WU, Yi-No, 242062 New Taipei City (TW); CHANG, Yu-Jen, 300193 Hsinchu City (TW); HSU, Wen-Chun, 242062 New Taipei City (TW); HWANG, Shiaw-Min, 22175 New Taipei City (TW); HUANG, Tzu-Hao, 22175 New Taipei City (TW)
(74) Representative: Straus, Alexander

(57) **Abstract**

Provided is a method for treating disorders or conditions related to erectile dysfunction. The method includes administering to a subject in need thereof a therapeutically effective amount of mesenchymal stem cells or secretome thereof, wherein the mesenchymal stem cells or secretome are derived from human amniotic fluid.

## Description

### Field of the Disclosure

Provided herein are methods of treatment and prevention of individuals having erectile dysfunction by mesenchymal stem cells (MSCs) or secretome thereof.

### Background of the Disclosure

Erectile function is a hemodynamic process of blood in-flow and pressure maintenance in the cavernosal spaces. Following sexual arousal and the release of nitric oxide to the erectile tissue, three processes occur to achieve an erection. These are relaxation of the trabecular smooth muscle, arterial dilation and venous compression. During the final stage, arterial flow fills sinusoidal spaces, compressing subtunical venules thereby reducing venous outflow. Blood flows into the cavernous spaces of the penis, thus expanding and stretching the penis into a rigid organ. The flow of blood in and out of the cavernous spaces is controlled by cavernous smooth muscle cells (CSMC) embedded in the trabeculae of the cavernous spaces.

Erectile dysfunction (ED) is a symptom that one of every ten men will suffer during his lifetime and ED may be associated with other problems that interfere with sexual intercourse, such as lack of desire and problems with orgasm and ejaculation.

Currently, ED may be treated with common oral medications including sildenafil, vardenafil, or tadalafil which may or may not improve ED. However, for people who are on medications that contain nitrates cannot take with these medications that may lead to side effects such as hypotension and these medications have many adverse reactions, which may include visual impairment, seizures, but not limited thereto.

Another treatment option of ED may include using a penis pump or a penile implant. However, as with any placement of devices during surgery, there is high risk of complications such as infection, bleeding, perforation of urethra, and penile numbness. Working alone, most drugs, pump and implants do not sufficiently allow the restore of ED and the treatment failure level is unacceptable high.

Cavernous nerve-sparing prostatectomy (CNSP) is a way to treat prostate diseases such as prostate cancer, which avoids cutting the nerve near the prostate. However, there are still problems regarding erectile function recovery effects and risk of infection after the CNSP. In some instances, even with very careful nerve-sparing techniques, based on variations in neuroanatomy among different patients, perfect execution of a given surgical technique may not be enough to accommodate to a specific patient's anatomy, resulting in the variability of erectile function recovery, and risk of leading to injury and degeneration of cavernous nerve is still quite high.

Accordingly, alternate systems and methods for treatment and prevention of individuals having erectile dysfunction to overcome the above-described drawbacks of the prior art are needed

### Summary of the Disclosure

In view of the above-described drawbacks, the present disclosure provides a method for treating a subject having erectile dysfunction, including administering to the subject a therapeutically effective amount of mesenchymal stem cells (MSCs). Also provided is a method for treating a subject having erectile dysfunction, including administering to the subject a therapeutically effective amount of secretome derived from mesenchymal stem cells.

In at least one embodiment of the present disclosure, the mesenchymal stem cells may be derived from amniotic fluid, bone marrow, umbilical cord blood, placental tissue, adipose tissue, peripheral blood, and dental pulp, but the present disclosure is not limited thereto. In some embodiments, the mesenchymal stem cells are preferably derived from human amniotic fluid.

In at least one embodiment of the present disclosure, the erectile dysfunction is caused by cardiovascular diseases, diabetes, anatomical defects, neurological problems, hormonal insufficiencies, drug side effects, or any combination thereof.

In at least one embodiment of the present disclosure, the erectile dysfunction is neurogenic erectile dysfunction.

In at least one embodiment of the present disclosure, the neurogenic erectile dysfunction is caused by stroke, brain and/or spinal injuries, diabetes, multiple sclerosis, Parkinson's disease, trauma from radical prostatectomy or radical pelvic surgeries, or any combination thereof.

In at least one embodiment of the present disclosure, the erectile dysfunction comprising a smooth muscle relation, an arterial dilation, a venous restriction, or neuronal atrophy.

In at least one embodiment of the present disclosure, the therapeutically effective amount of the mesenchymal stem cells is at least 1x10⁶. In some embodiments, the therapeutically effective amount of the mesenchymal stem cells derived from the amniotic fluid is administered to the subject from about 1x10⁶ to 2*10⁸ to improve smooth muscle relation, intracorporal pressure, an arterial dilation, a venous restriction or neuronal atrophy

In at least one embodiment of the present disclosure, the mesenchymal stem cells may be proliferated in culture for a period of at least 2, 3, or 4 weeks.

In at least one embodiment of the present disclosure, the mesenchymal stem cells are positive for CD 73, CD 90, CD 105, Nestin, Sox2, or any combination thereof, and mesenchymal stem cells are negative for CD 34, CD 45, CD 14, CD 11b, CD 79α, CD 19, HLA-DR, or any combination thereof.

In at least one embodiment of the present disclosure, the mesenchymal stem cells may have, but not limited to, a spindle-shaped morphology, flattened morphology, or fibroblast-like morphology in attachment culture.

In at least one embodiment of the present disclosure, the mesenchymal stem cells have osteogenic differentiability, adipogenic differentiability, chondrogenic differentiability, or any combination thereof.

In at least one embodiment of the present disclosure, the mesenchymal stem cells are obtained by steps comprising:
(a) obtaining an amniotic fluid sample from first trimester amniotic fluid, second trimester or third trimester amniotic fluid by amniocentesis or caesarean section;
(b) centrifuging the amniotic fluid sample at 200xg for at least 5 minutes and removing supernatants;
(c) culturing cells with α-modified minimum essential medium supplemented with 1-5% human platelet lysate or 10 to 20% fetal bovine serum or using commercial available mesenchymal stem cell medium;
(d) removing non-adherent cells after 2 to 4 days of culture; and let the adherent cells grow as colonies for the following 7 to 14 days;
(e) trypsinizing adherent cells and passing the cells at a seeding density of 1000 to 9000 cells/cm² for expansion.

In some embodiments of the present disclosure, the α-modified minimum essential medium comprises 1 to 20 ng/ml basic fibroblast growth factor, e.g., about 1, 4, 10 or 20 ng/ml basic fibroblast growth factor. In some embodiments of the present disclosure, the α-modified minimum essential medium does not comprise the basic fibroblast growth factor.

In at least one embodiment of the present disclosure, the secretome of mesenchymal stem cells is obtained by steps comprising:
(a) culturing amniotic fluid stem cells in a basal medium for about 24 to 72 hours when the cells reached about 80% confluence;
(b) collecting a supernatant of the culture medium after centrifuging at about 300 xg for about 10 minutes to eliminate dead cells and debris; and
(c) filtering the supernatant with a 0.22-µm filter and storing the secretome at about -20 °C.

Stem cell therapy has been proposed for the treatment of ED as stem cells may differentiate to endothelial, neuronal or smooth muscle cells and therefore restore possible structural damage in the penile tissue. The use of the mesenchymal stem cells obtained from amniotic fluid may regenerate and promote the propagation and differentiation of progenitor cells, thus improving the recovery of the target tissue of the penis via a local or systemic injection. Therefore, the mesenchymal stem cells of amniotic fluid are used to improve or to treat the condition of erectile dysfunction. Compared with the prior art, when the mesenchymal stem cells of amniotic fluid of the present disclosure is applied to a subject, as the mesenchymal stem cells of amniotic fluid may differentiate to endothelial, neuronal or smooth muscle cells and therefore restore possible structural damage in the penile tissue, therefore, at least the erectile ability, actin expression of smooth muscle of corpus cavernosum tissue, structure of the cavernous nerve, and vWF expression of corpus cavernosum tissue may be greatly improved, which is useful for treating ED.

### Brief Description of the Drawings

The patent or application file contains at least one drawing executed in color. Copies of this patent or patent application publication with color drawing(s) will be provided by the Office upon request and payment of the necessary fee.

The present disclosure will become more readily appreciated by reference to the following descriptions in conjunction with the accompanying drawings.

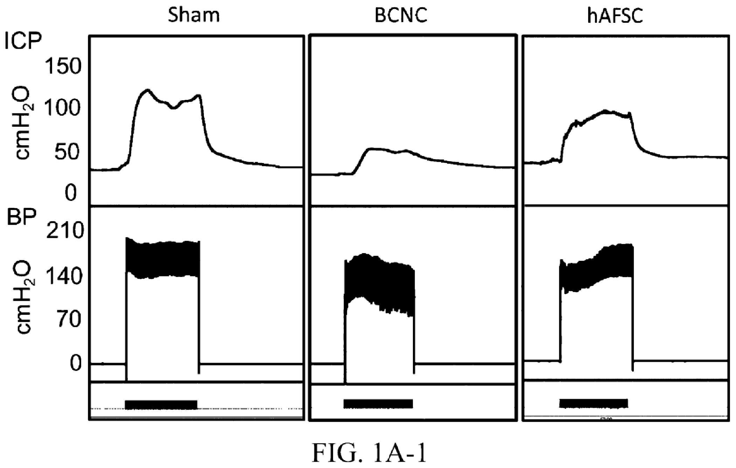

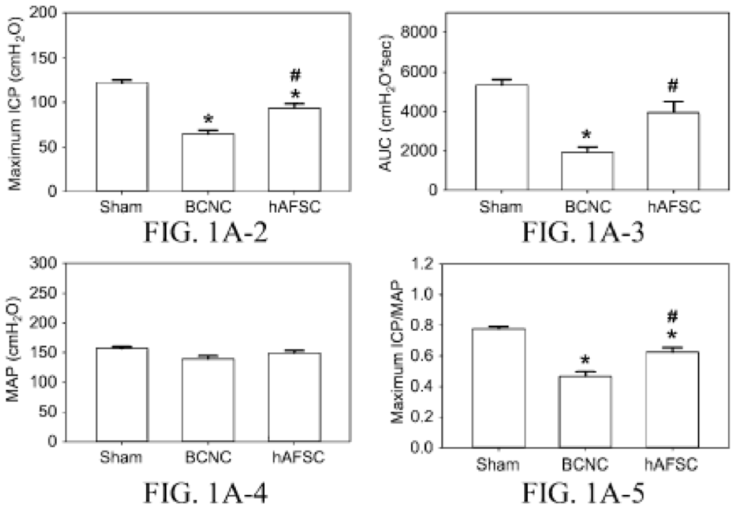

FIG. 1A-1 is a typical example of recording of intracavernosal pressure (ICP) and mean arterial blood pressure (BP) in the responses to electrostimulation of distal end of the cavernous nerve in the sham group, bilateral cavernous nerve crushing (BCNC) injury group and human amniotic fluid stem cell (hAFSC) group. The x-axis represents time in seconds, and the green bar represents one electrical stimulus of 60 seconds. The y-axis represents the ICP and BP (top and bottom panels) in experimental animals.
FIG. 1A-2 is a measurement of maximum ICP (cmH₂O) in the sham group, BCNC group and hAFSC group.
FIG. 1A-3 is a measurement of Area Under Curve (AUC, cmH₂O*sec) in the sham group, BCNC group and hAFSC group.
FIG. 1A-4 is a measurement of mean arterial pressure (MAP, cmH₂O) in the sham group, BCNC group and hAFSC group.
FIG. 1A-5 is a measurement of maximum ICP/MAP in the sham group, BCNC group and hAFSC group.
FIG. 1B-1 is a typical example of recording of ICP and mean arterial blood pressure (BP) in the responses to electrostimulation of distal end of the cavernous nerve in the sham group, BCNC group and BCNC treated with secretome of hAFSC group. The x-axis represents time in seconds, and the green bar represents one electrical stimulus of 60 seconds. The y-axis represents the ICP and BP (top and bottom panels) in experimental animals.

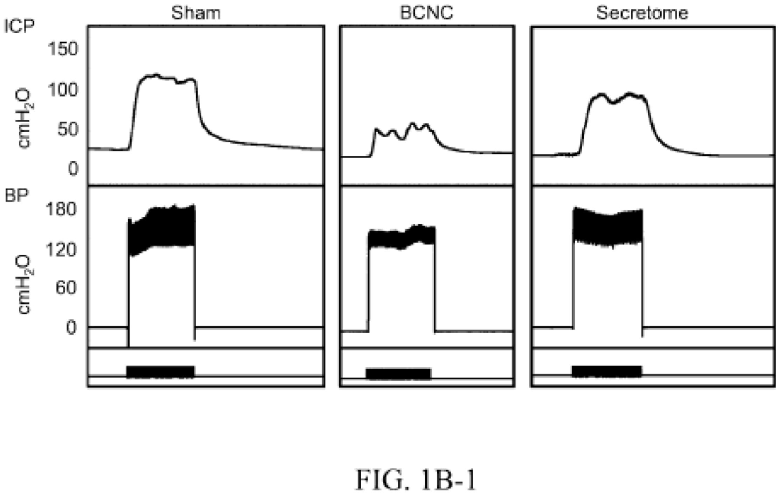

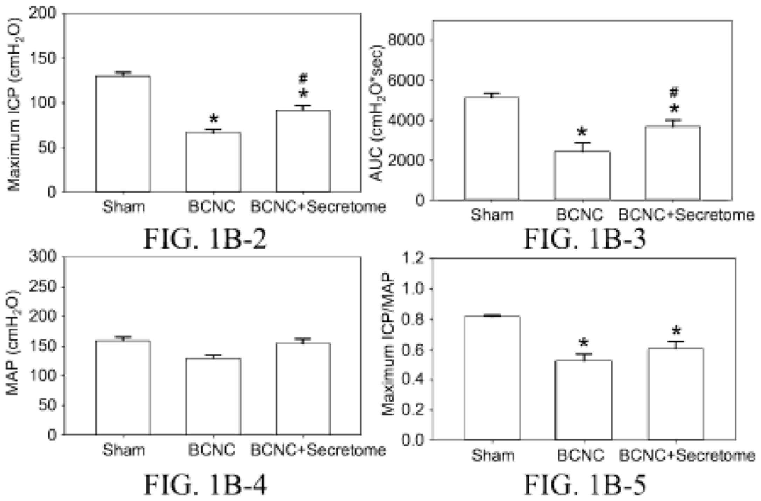

FIG. 1B-2 is a measurement of maximum ICP (cmH₂O) in the sham group, BCNC group, and BCNC treated with secretome of hAFSC group.
FIG. 1B-3 is a measurement of AUC (cmH₂O*sec) in the sham group, BCNC group, and BCNC treated with secretome of hAFSC group.
FIG. 1B-4 is a measurement of MAP (cmH₂O) in the sham group, BCNC group, and BCNC treated with secretome of hAFSC group.
FIG. 1B-5 is a measurement of maximum ICP/MAP in the sham group, BCNC group, and BCNC treated with secretome of hAFSC group.

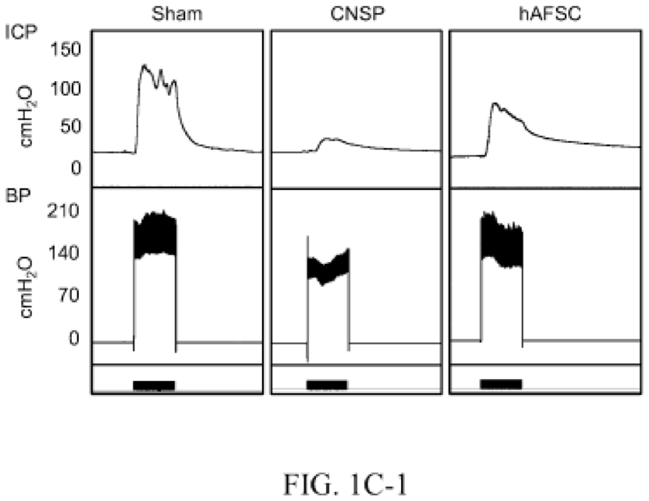

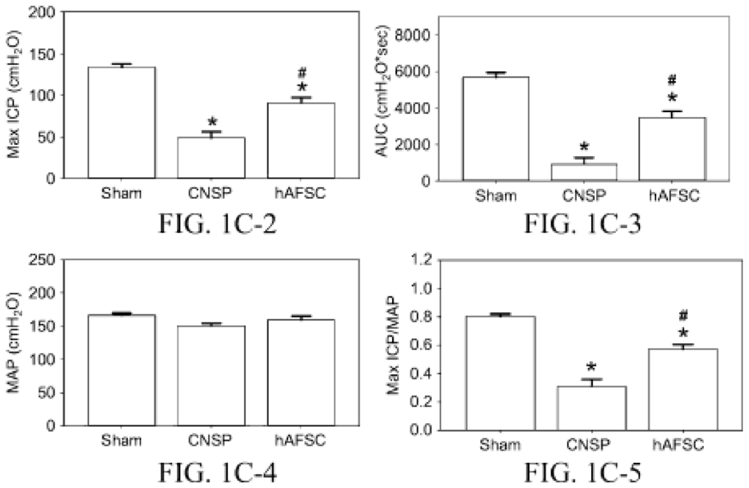

FIG. 1C-1 is a typical example of recording of ICP and mean arterial blood pressure (BP) in the responses to electrostimulation of distal end of the cavernous nerve in the sham group, CNSP group and CNSP treated with hAFSC group. The x-axis represents time in seconds, and the green bar represents one electrical stimulus of 60 seconds. The y-axis represents the ICP and BP (top and bottom panels) in experimental animals.
FIG. 1C-2 is a measurement of maximum ICP (cmH₂O) in the sham group, CNSP group, and CNSP treated with hAFSC group.
FIG. 1C-3 is a measurement of AUC (cmH₂O*sec) in the sham group, CNSP group, and CNSP treated with hAFSC group.
FIG. 1C-4 is a measurement of MAP (cmH₂O) in the sham group, CNSP group, and CNSP treated with hAFSC group.
FIG. 1C-5 is a measurement of maximum ICP/MAP in the sham group, CNSP group, and CNSP treated with hAFSC group.

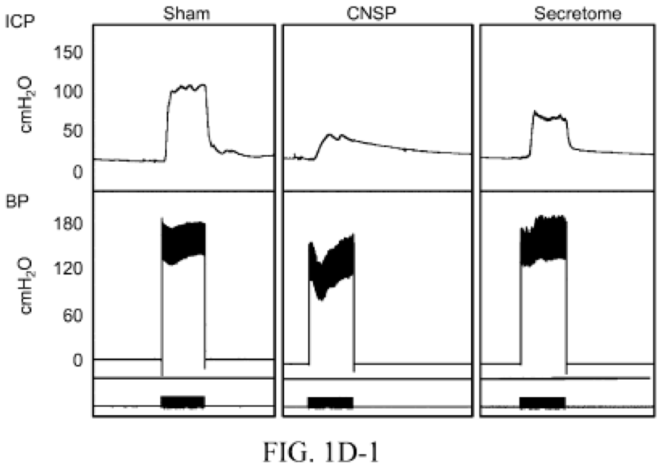

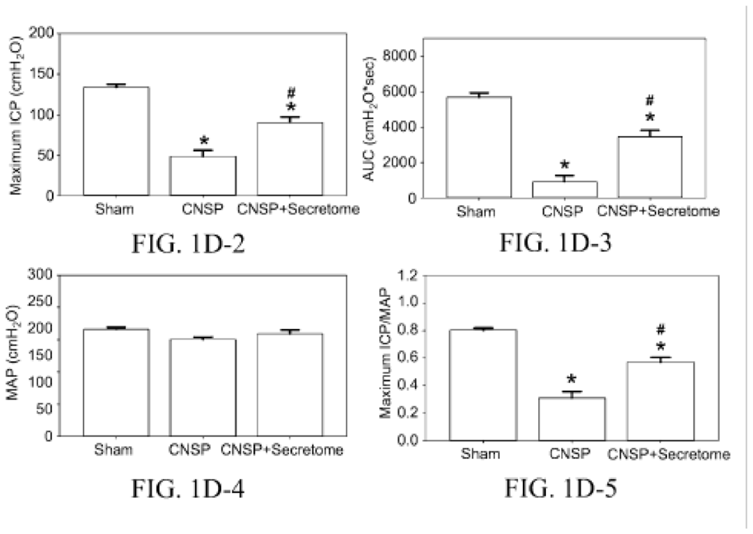

FIG. 1D-1 is a typical example of recording of ICP and BP in the responses to electrostimulation of distal end of the cavernous nerve in the sham group, CNSP group and CNSP treated with secretome of hAFSC group. The x-axis represents time in seconds, and the green bar represents one electrical stimulus of 60 seconds. The y-axis represents the ICP and BP (top and bottom panels) in experimental animals.
FIG. 1D-2 is a measurement of maximum ICP (cmH₂O) in the sham group, CNSP group, and CNSP treated with secretome of hAFSC group.
FIG. 1D-3 is a measurement of AUC (cmH₂O*sec) in the sham group, CNSP group, and CNSP treated with secretome of hAFSC group.
FIG. 1D-4 is a measurement of MAP (cmH₂O) in the sham group, CNSP group, and CNSP treated with secretome of hAFSC group.
FIG. 1D-5 is a measurement of maximum ICP/MAP in the sham group, CNSP group, and CNSP treated with secretome of hAFSC group.

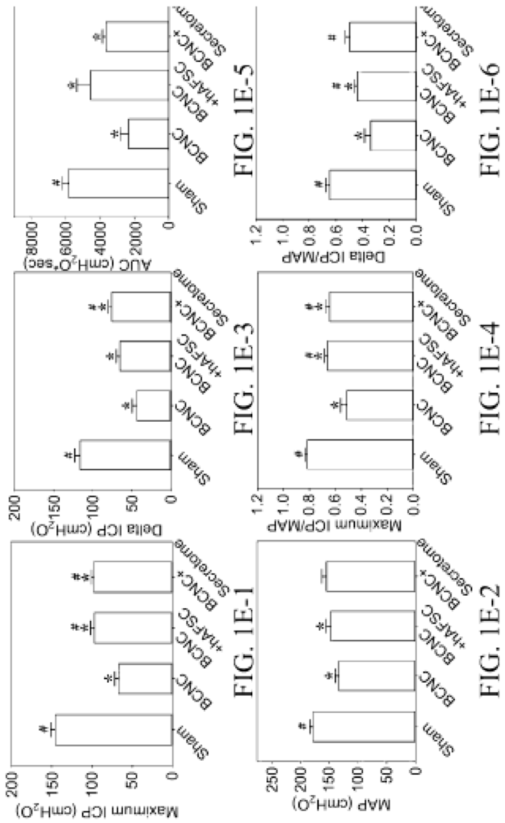

FIG. 1E-1 is a measurement of maximum ICP (cmH₂O) in the sham group, BCNC group, BCNC treated with hAFSC group and BCNC treated with secretome of hAFSC group.
FIG. 1E-2 is a measurement of MAP (cmH₂O) in the sham group, BCNC group, BCNC treated with hAFSC group and BCNC treated with secretome of hAFSC group.
FIG. 1E-3 is a measurement of Delta ICP (cmH₂O) in the sham group, BCNC group, BCNC treated with hAFSC group and BCNC treated with secretome of hAFSC group.
FIG. 1E-4 is a measurement of Maximum ICP/MAP in the sham group, BCNC group, BCNC treated with hAFSC group and BCNC treated with secretome of hAFSC group.
FIG. 1E-5 is a measurement of AUC (cmH₂O*sec) in the sham group, BCNC group, BCNC treated with hAFSC group and BCNC treated with secretome of hAFSC group.
FIG. 1E-6 is a measurement of Delta ICP/MAP in the sham group, BCNC group, BCNC treated with hAFSC group and BCNC treated with secretome of hAFSC group.

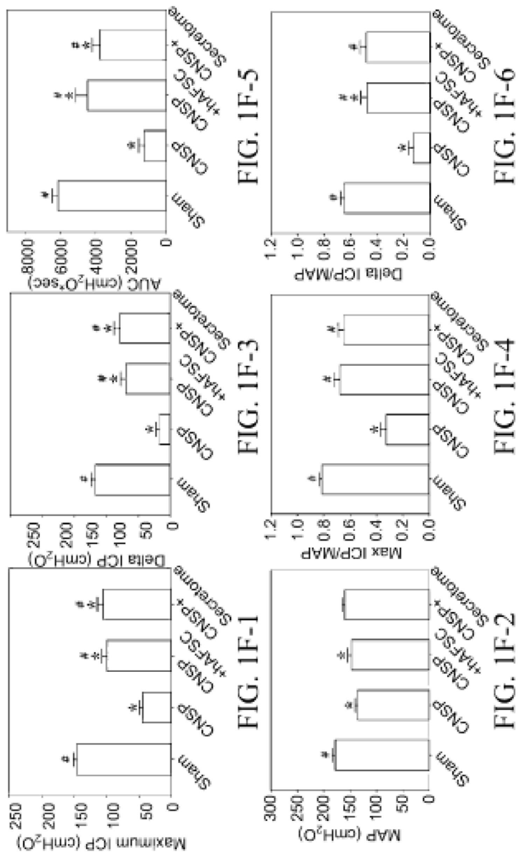

FIG. 1F-1 is a measurement of maximum ICP (cmH₂O) in the sham group, CNSP group, CNSP treated with hAFSC group and CNSP treated with secretome of hAFSC group.
FIG. 1F-2 is a measurement of MAP (cmH₂O) in the sham group, CNSP group, CNSP treated with hAFSC group and CNSP treated with secretome of hAFSC group.
FIG. 1F-3 is a measurement of Delta ICP (cmH₂O) in the sham group, CNSP group, CNSP treated with hAFSC group and CNSP treated with secretome of hAFSC group.
FIG. 1F-4 is a measurement of Maximum ICP/MAP in the sham group, CNSP group, CNSP treated with hAFSC group and CNSP treated with secretome of hAFSC group.
FIG. 1F-5 is a measurement of AUC (cmH₂O*sec) in the sham group, CNSP group, CNSP treated with hAFSC group and CNSP treated with secretome of hAFSC group.
FIG. 1F-6 is a measurement of Delta ICP/MAP in the sham group, CNSP group, CNSP treated with hAFSC group and CNSP treated with secretome of hAFSC group.

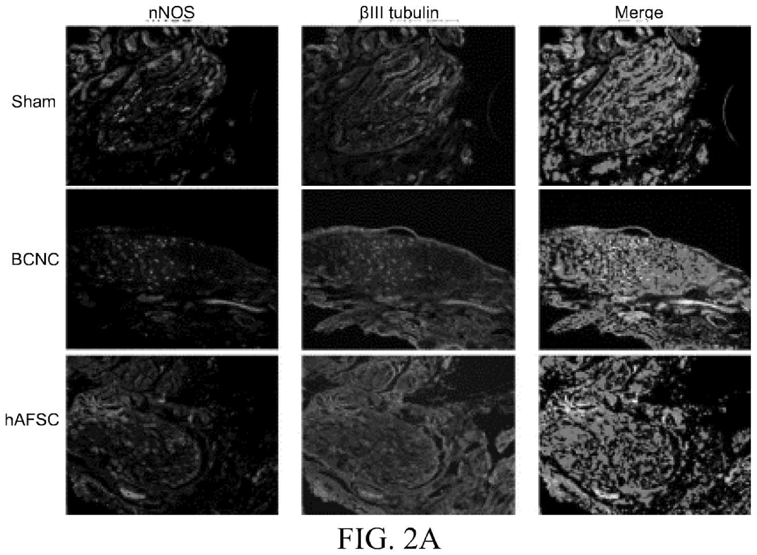

FIG. 2A is an immunofluorescence staining of nNOS, and β-III tubulin in the major pelvic ganglion of the sham group, BCNC group and hAFSC group.

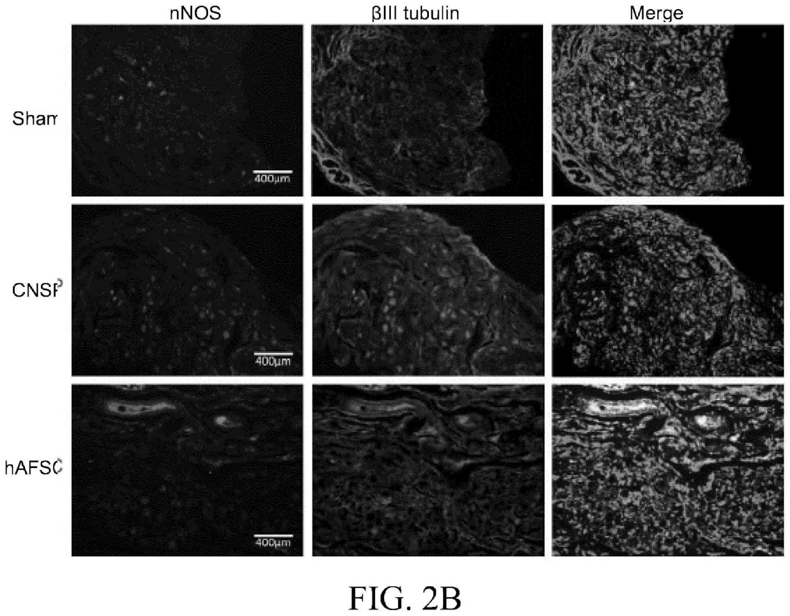

FIG. 2B is an immunofluorescence staining of nNOS, and β-III tubulin in major pelvic ganglion of the sham group, CNSP group and hAFSC group. Scale bar =400µm.

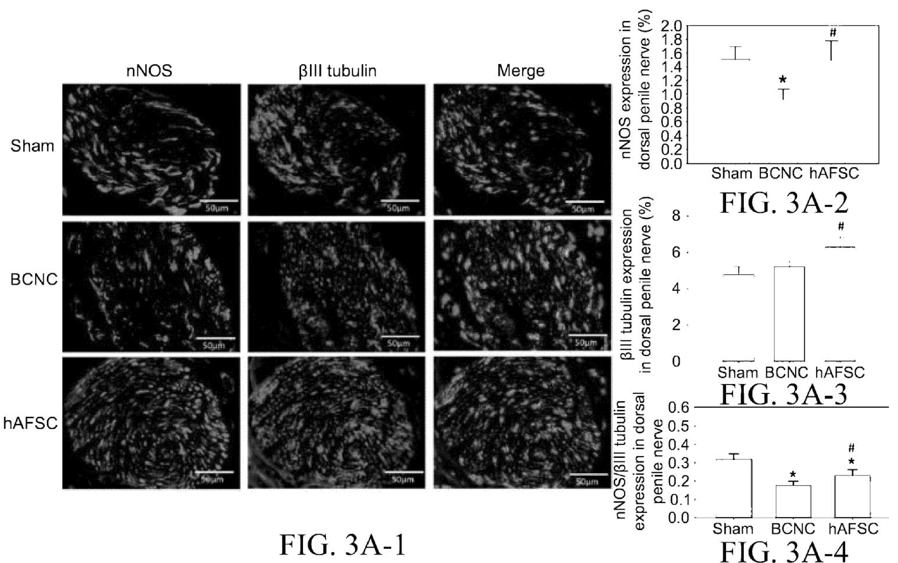

FIG. 3A-1 is an immunofluorescence staining of nNOS and β-III tubulin in dorsal penile nerve of the sham group, BCNC group and hAFSC group. Scale bar = 50 µm.
FIG. 3A-2 shows nNOS expression level in dosral penile nerve (%) of the sham group, BCNC group and hAFSC group.
FIG. 3A-3 shows β-III tubulin expression level in dorsal penile nerve (%) of the sham group, BCNC group and hAFSC group.
FIG. 3A-4 is a ratio of nNOS/ β-III tubulin expression in dorsal penile nerve of the sham group, BCNC group and hAFSC group.

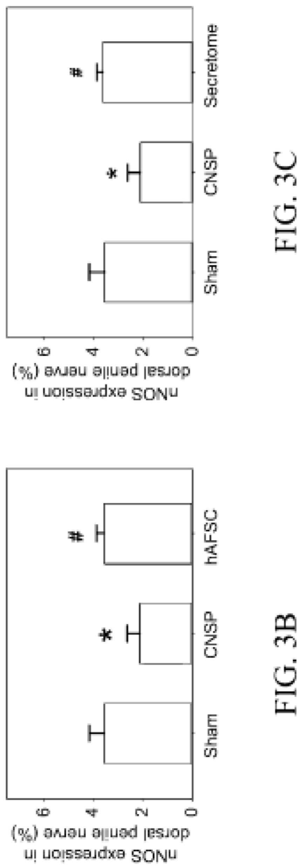

FIG. 3B shows nNOS expression level in dorsal penile nerve (%) of the sham group, CNSP group and hAFSC group.
FIG. 3C shows nNOS expression level in dorsal penile nerve (%) in the sham group, CNSP group and secretome of hAFSC group.

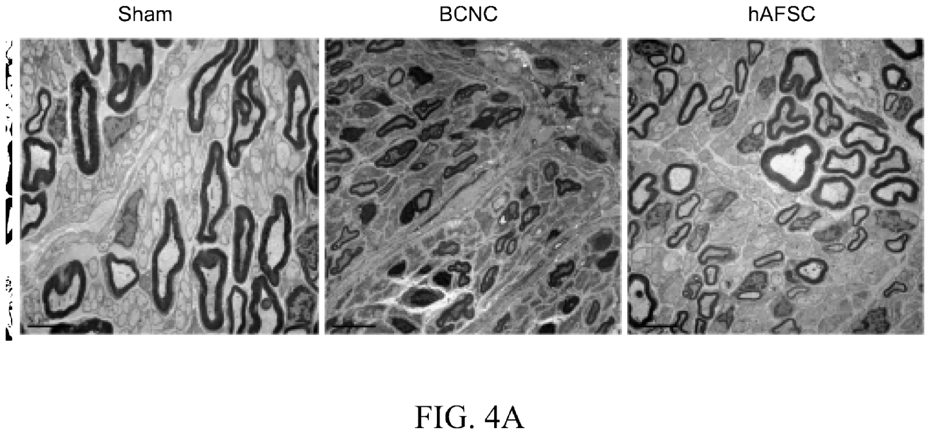

FIG. 4A is an ultrastructural analysis of the cavernous nerve in sham group, BCNC group and hAFSC group conducted by transmission electron microscopy (TEM).

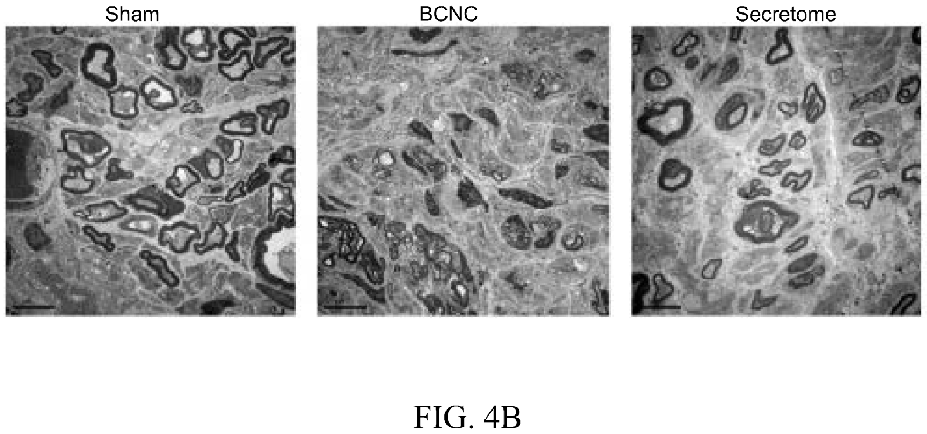

FIG. 4B is an ultrastructural analysis of the cavernous nerve in sham group, BCNC group and secretome of hAFSC group conducted by TEM.

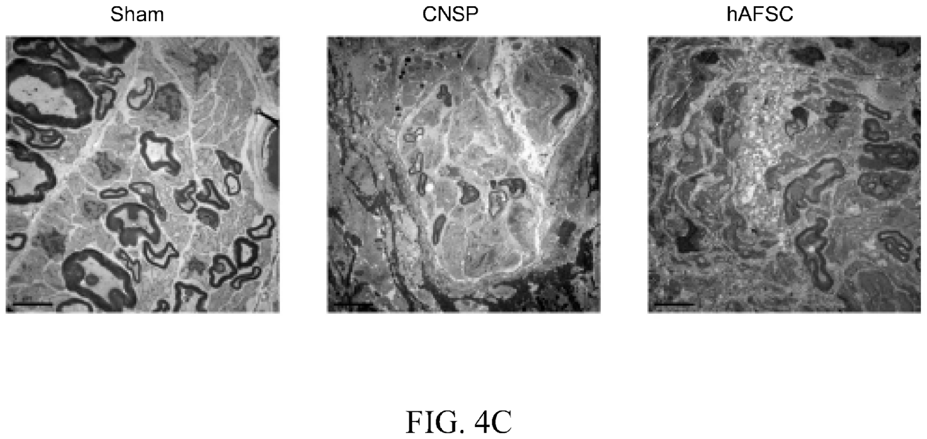

FIG. 4C is an ultrastructural analysis of the cavernous nerve in sham group, CNSP group and hAFSC group conducted by TEM.

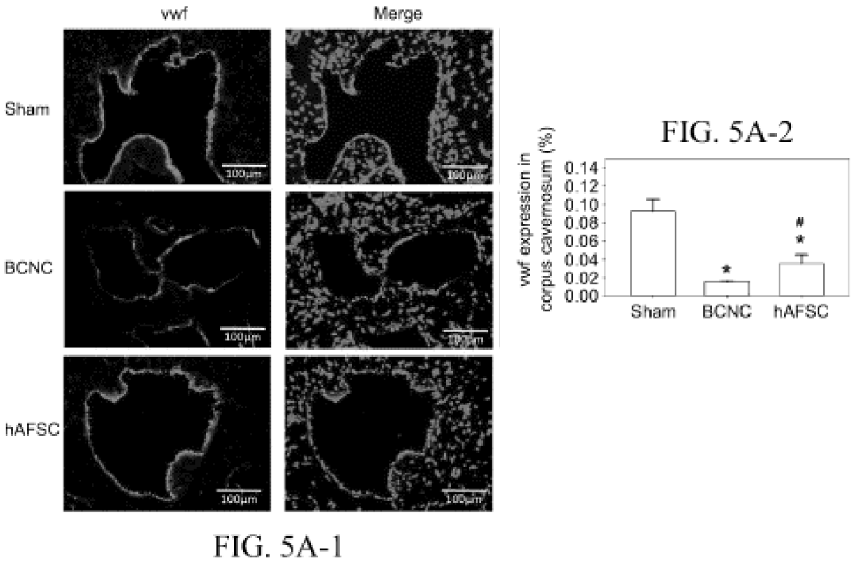

FIG. 5A-1 is a set of immunofluorescence staining images of vWF of corpus cavernosum in sham group, BCNC group and hAFSC group. Characteristics of sham, BCNC and hAFSC via staining of the cell-specific marker vWF and merged immunostainings are provided. vWF are represented by green color, and the nucleus is labeled by blue color. The stain at higher intensity is due to the presence of vWF in the corpus cavernosum. Scale bar = 100 µm.
FIG. 5A-2 is a graph shows the quantification of vWF expression of corpus cavernosum (%) in the sham group, BCNC group and hAFSC group.

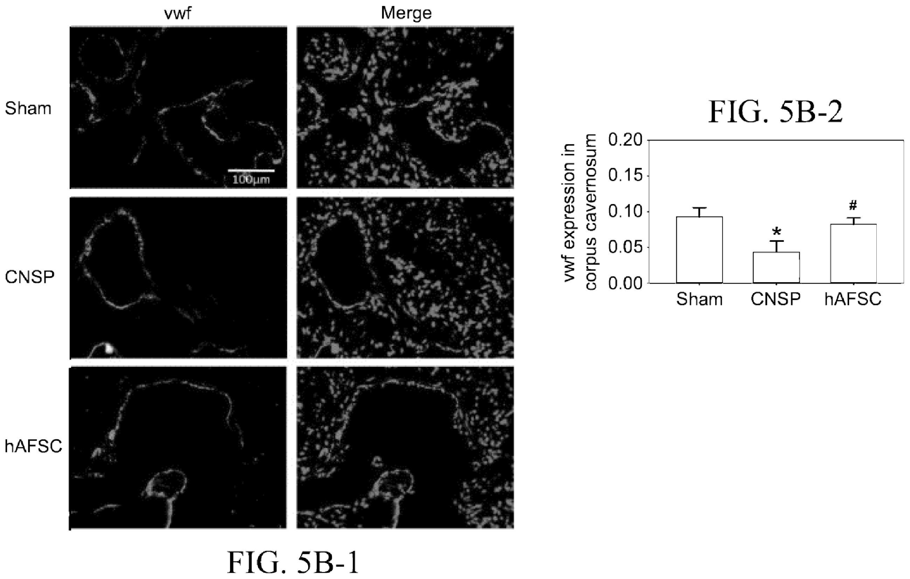

FIG. 5B-1 is a set of immunofluorescence staining images of vWF of corpus cavernosum in sham group, CNSP group, and hAFSC group. Scale bar = 100 µm.
FIG. 5B-2 is a graph shows the quantification of vWF expression of corpus cavernosum in the sham group, CNSP group and hAFSC group.

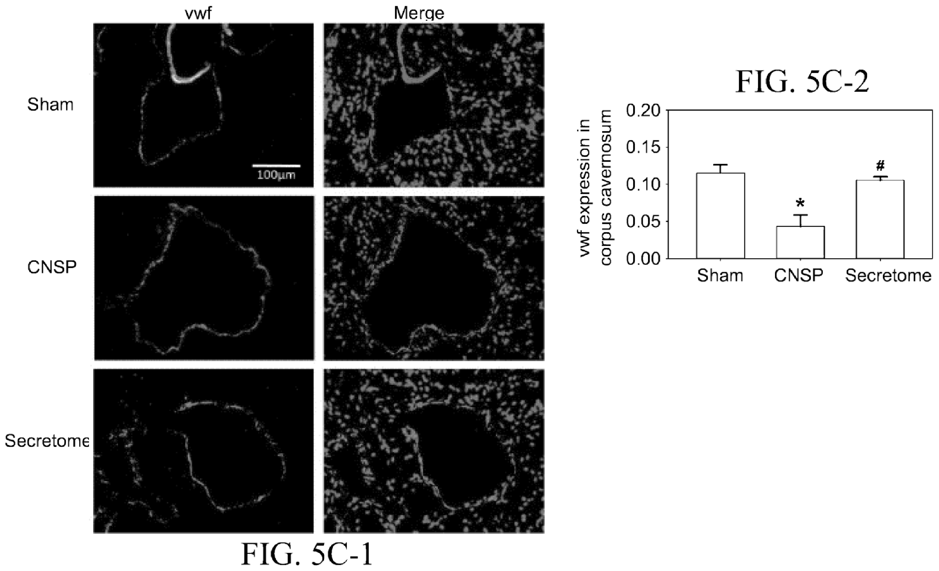

FIG. 5C-1 is a set of immunofluorescence staining images of vWF of corpus cavernosum in sham group, CNSP group, and Secretome of hAFSC group. Characteristics of sham, CNSP and secretome of hAFSC via staining of the cell-specific marker vWF (light green) and merged immunostainings are provided. Secretome of hAFSC groups showed significantly a higher of vWF expression compared to the CNSP group. Scale bar = 100 µm.
FIG. 5C-2 is graph shows the quantification of vWF expression of corpus cavernosum (%) in the sham group, CNSP group and secretome of hAFSC group.

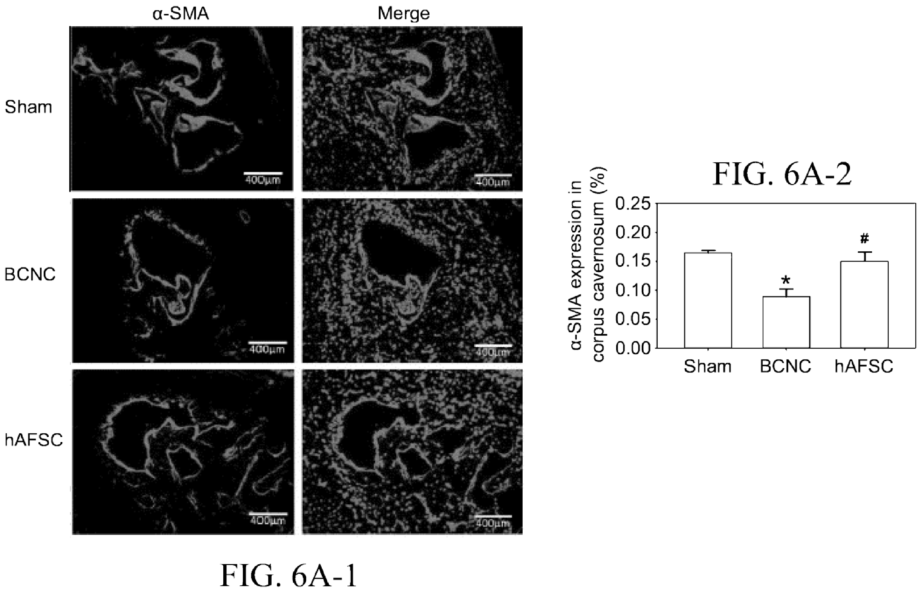

FIG. 6A-1 is a set of immunofluorescences staining images of α-smooth muscle actin expression of corpus cavernosum in the sham group, BCNC group, and hAFSC group. Scale bar = 400 µm.
FIG. 6A-2 is a measurement of α-smooth muscle actin (α-SMA) expression of corpus cavernosum of sham group, BCNC group, and hAFSC group.

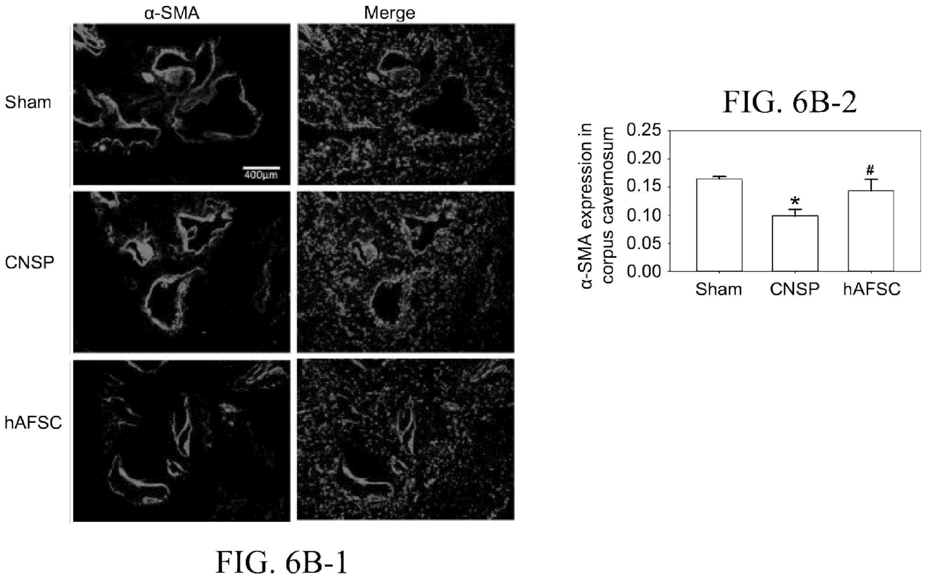

FIG. 6B-1 is a set of immunofluorescences staining images of α-smooth muscle actin expression in corpus cavernosum of the sham group, CNSP group, and hAFSC group. Scale bar = 400 µm.
FIG. 6B-2 is a measurement of α-SMA expression in corpus cavernosum of sham group, CNSP group, and hAFSC group.

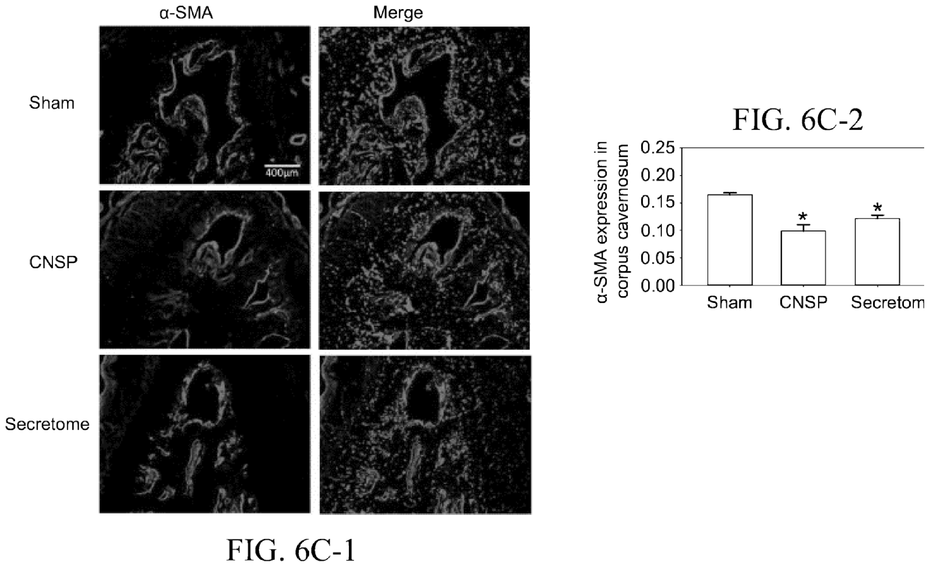

FIG. 6C-1 is a set of immunofluorescences staining images of α-smooth muscle actin in corpus cavernosum of the sham group, CNSP group, and secretome of hAFSC group. Scale bar = 400 µm.
FIG. 6C-2 is a measurement of α-SMA expression in corpus cavernosum of sham group, CNSP group, and Secretome of hAFSC group.

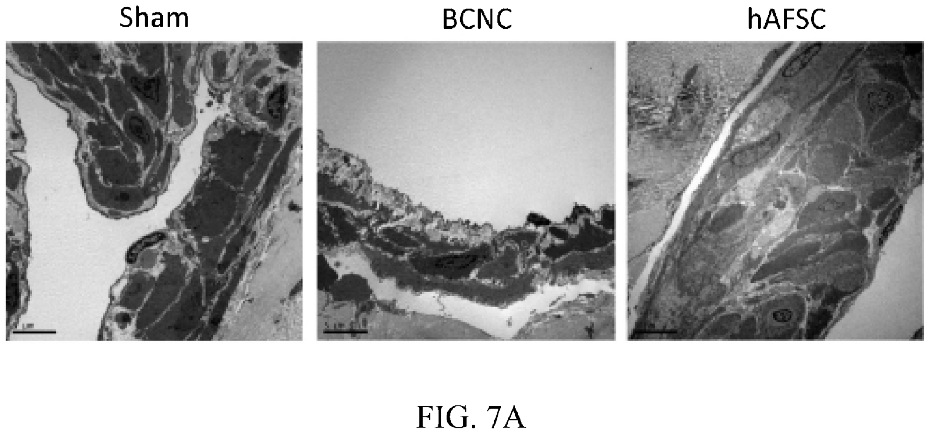

FIG. 7A is an ultrastructural analysis of the corpus cavernosum (CC) tissue in the sham group, BCNC group and hAFSC group conducted by transmission electron microscopy (TEM). The TEM images revealed a thicker and tighter muscle layers in sham and hAFSC group, comparing to the BCNC group. Scale bar = 5 µm.

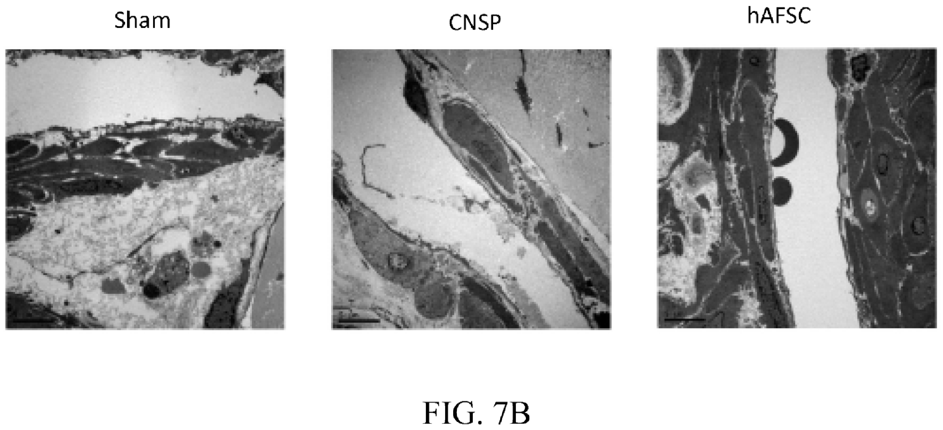

FIG. 7B is an ultrastructural analysis of the corpus cavernosum (CC) tissue in the sham group, CNSP group and hAFSC group conducted by TEM. The TEM images revealed a thicker and tighter muscle layers in Sham and hAFSC group, comparing to the CNSP group. Scale bar = 5 µm.

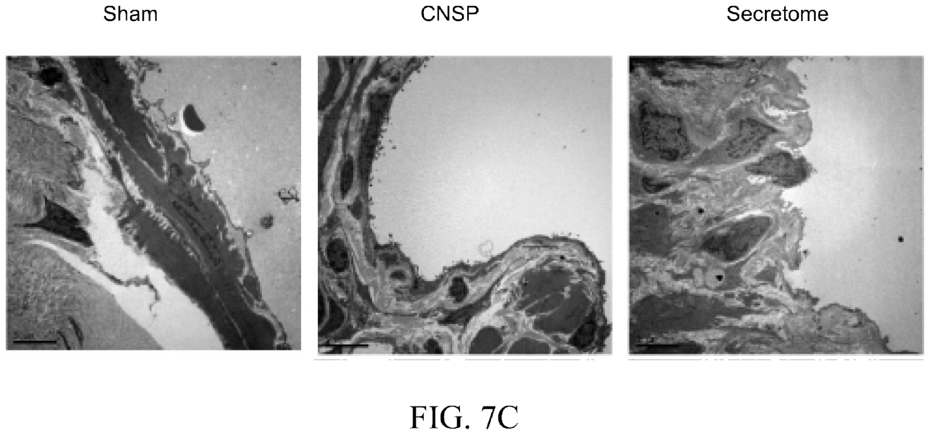

FIG. 7C is an ultrastructural analysis of the corpus cavernosum (CC) tissue in the sham group, CNSP group and secretome of hAFSC group conducted by TEM. The TEM images revealed a thicker and tighter muscle layers in sham and secretome of hAFSC group, comparing to the CNSP group. Scale bar = 5 µm.

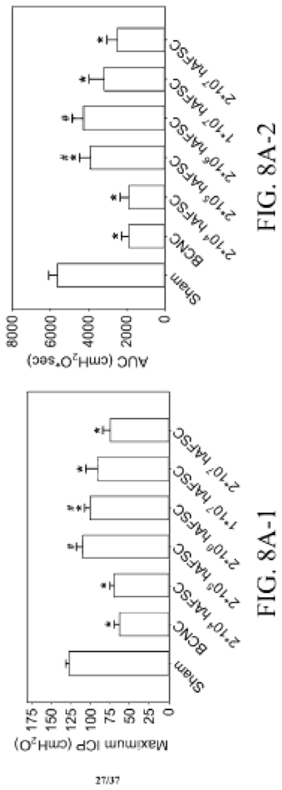

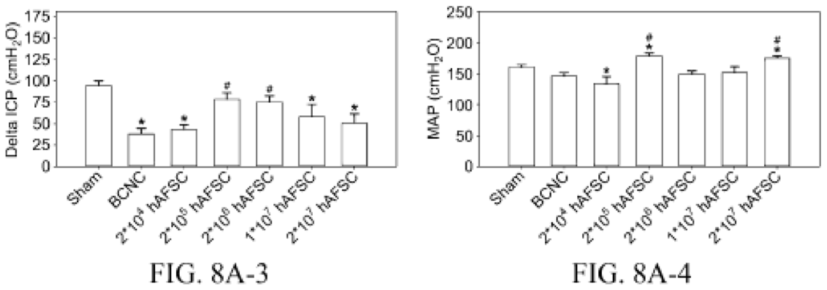

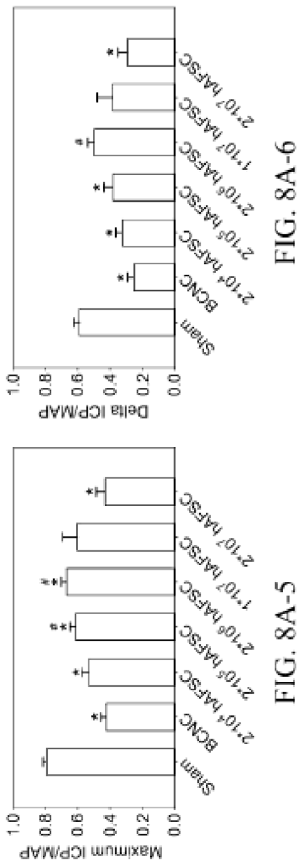

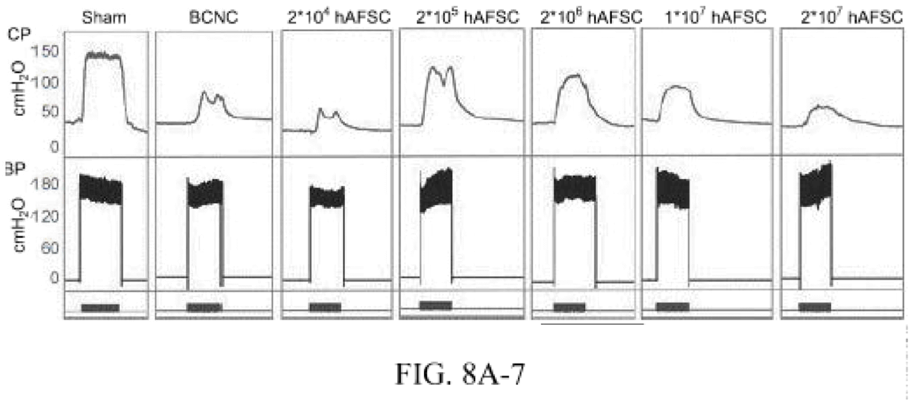

FIG. 8A-1 is a measurement of maximum ICP (cmH₂O) in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-2 is a measurement of Area Under Curve (AUC, cmH₂O*sec) in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-3 is a change of ICP (cmH2O) in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-4 is a measurement of mean arterial pressure (MAP, cmH₂O) in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-5 is a measurement of maximum ICP/MAP in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-6 is a change of ICP/MAP in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.
FIG. 8A-7 is a typical example of recording of intracavernosal pressure (ICP) and mean arterial blood pressure (BP) in the responses to electrostimulation of distal end of the cavernous nerve in the sham group, bilateral cavernous nerve crushing (BCNC) injury group, and human amniotic fluid stem cell (hAFSC) groups in various concentrations including 2*10⁴ hAFSC, 2*10⁵ hAFSC, 2*10⁶ hAFSC, 1*10⁷ hAFSC, and 2*10⁷ hAFSC. The x-axis represents time in seconds, and the green bar represents one electrical stimulus of 60 seconds. The y-axis represents the ICP and BP (top and bottom panels) in experimental animals.

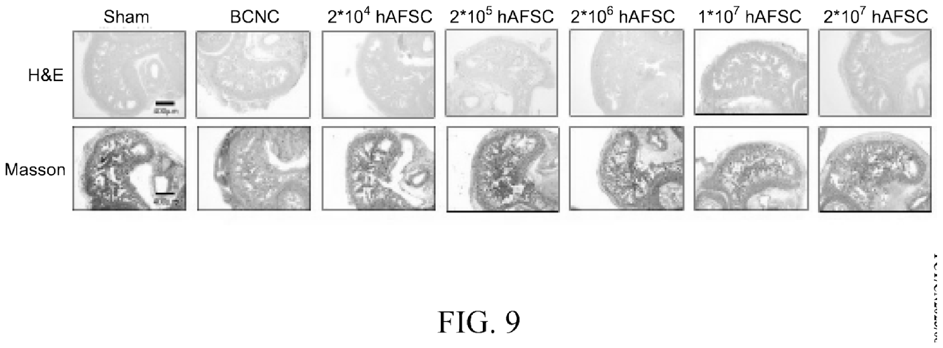

FIG. 9 includes H&E staining and Masson's trichrome staining of the cavernous nerve samples in sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC. Scale bar = 400 µm.

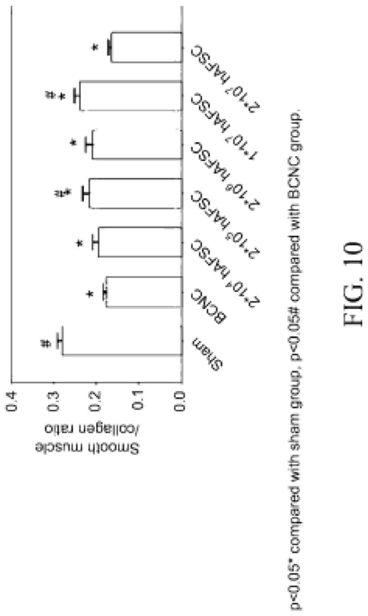

FIG. 10 is a measurement of smooth muscle to collagen ratio in the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC. p<0.05* compared with sham group, p<0.05# compared with BCNC group.

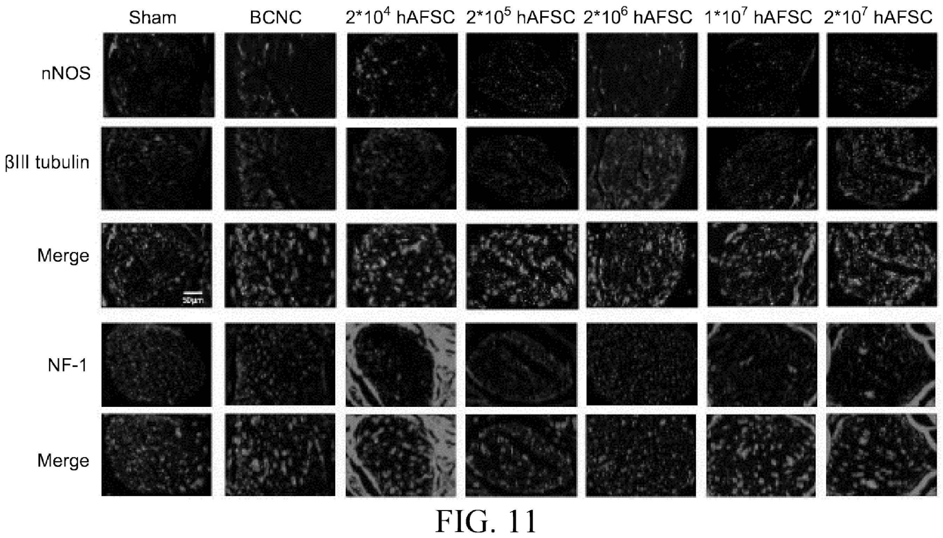

FIG. 11 is an immunofluorescence staining of nNOS, β-III tubulin, NF-1 images are provided in dorsal penile nerve of the sham group, BCNC group and hAFSC groups in various concentrations including 2*10⁴ hAFSC, 2*10⁵ hAFSC, 2*10⁶ hAFSC, 1*10⁷ hAFSC, and 2*10⁷ hAFSC. Scale bar = 50 µm. nNOS is represented by green color. β-III tubulin and NF-1 are represented by red color, and the nucleus is labeled by blue color.

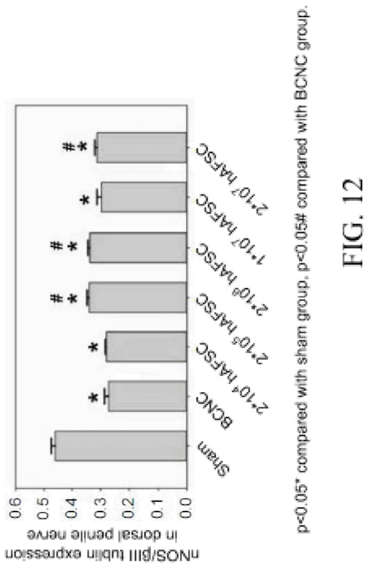

FIG. 12 is a ratio of nNOS/ β-III tubulin expression in dorsal penile nerve of the sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.

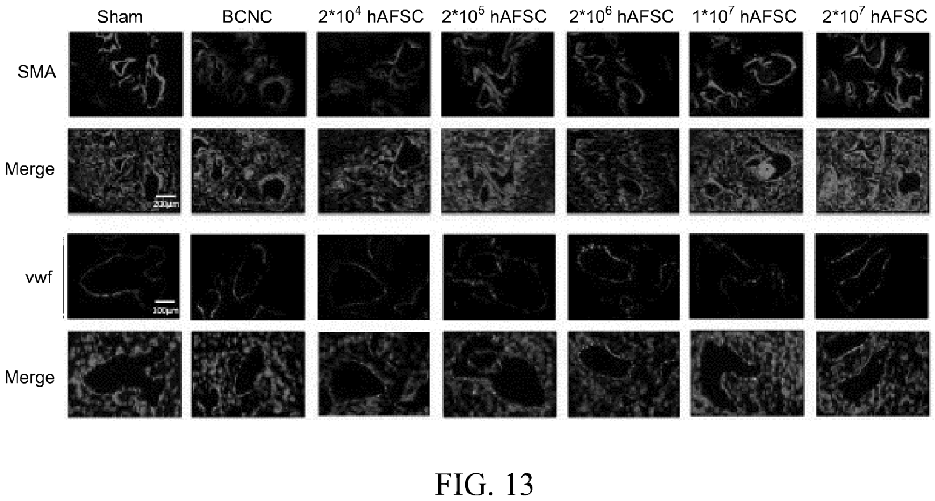

FIG. 13 is a set of immunofluorescence staining images of α-SMA and vWf of corpus cavernosum in sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC. α-smooth muscle actin (α-SMA) is represented by red color. vWf are represented by green color, and the nucleus is labeled by blue color. Scale bar of α-SMA= 200 µm. Scale bar of vWF= 100 µm

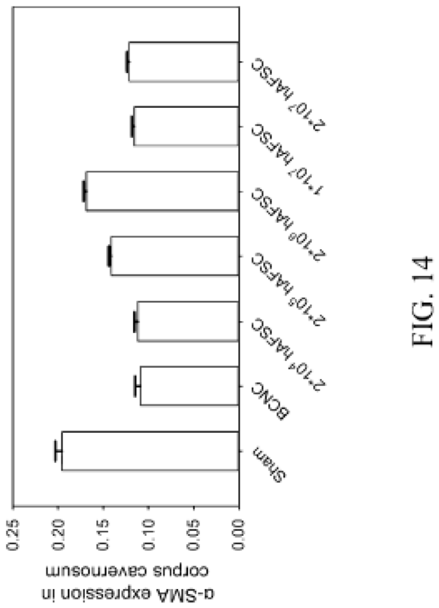

FIG. 14 is a measurement of α-SMA expression in corpus cavernosum of sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC.

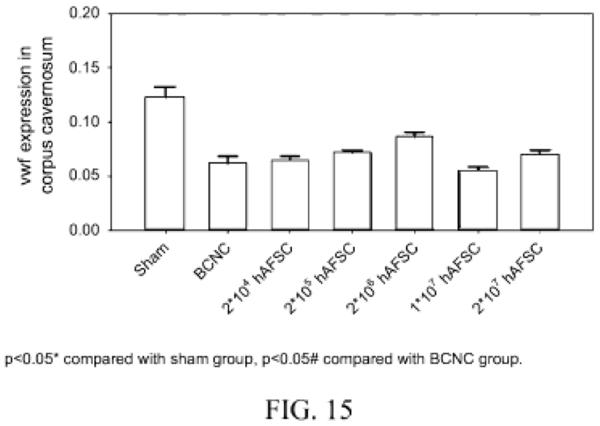

FIG. 15 is a measurement of vWf expression in corpus cavernosum of sham group, BCNC group, and hAFSC groups in various concentrations including 2*10⁴ hAFSC, 2*10⁵ hAFSC, 2*10⁶ hAFSC, 1*10⁷ hAFSC, and 2*10⁷ hAFSC. p<0.05* compared with sham group, p<0.05# compared with BCNC group.

### Detailed Description

The following embodiments are provided to illustrate the present disclosure in detail. A person having ordinary skill in the art can easily understand the advantages and effects of the present disclosure after reading the disclosure of this specification, and also can implement or apply in other different embodiments. Therefore, it is possible to modify and/or alter the following embodiments for carrying out this disclosure without contravening its scope for different aspects and applications, and any element or method within the scope of the present disclosure disclosed herein can combine with any other element or method disclosed in any embodiments of the present disclosure.

As used herein, the singular forms "a," "an," and "the" include plural referents unless expressly and unequivocally limited to one referent. The term "or" is used interchangeably with the term "and/or" unless the context clearly indicates otherwise.

The numeral ranges used herein are inclusive and combinable, any numeral value that falls within the numeral scope herein could be taken as a maximum or minimum value to derive the sub-ranges therefrom. For example, it should be understood that the numeral range "10 to 20%" comprises any sub-ranges between the minimum value of 10% to the maximum value of 20%, such as the sub-ranges from 10% to 15%, from 15% to 20%, and from 12.5% to 17.5%.

The term "about" as used herein when referring to the numerical value is meant to encompass variations of ±20%, ±10%, ±5%, ±1%, ±0.5%, or ±0.1% from the numerical value. Such variations in the numerical value may occur by, e.g., the experimental error, the typical error in measuring or handling procedure for making compounds, compositions, concentrates, or formulations, the differences in the source, manufacture, or purity of starting materials or ingredients used in the present disclosure, or like considerations.

As used herein, "subject" may encompass any vertebrate including, but not limited to, humans, mammals, reptiles, amphibians, and/or fish. However, advantageously, the subject is a mammal such as a human, or an animal mammal such as a domesticated mammal, e.g., a dog, a cat, a horse, a rat, a mouse, or the like, or a production mammal, e.g., a cow, a sheep, a pig, or the like.

As used herein, the terms "comprise," "comprising," "include," "including," "have," "having," "contain," "containing," and any other variations thereof are intended to cover a non-exclusive inclusion. For example, when describing an object "comprises" a limitation, unless otherwise specified, it may additionally include other ingredients, elements, components, structures, regions, parts, devices, systems, steps, or connections, etc., and should not exclude other limitations.

As used herein "erectile dysfunction" refers to the persistent inability to attain and maintain an erection sufficient to permit satisfactory sexual performance. The etiological factors of neurogenic ED may include, but not limited to, stroke, brain and spinal injury, type I and II diabetes mellitus, chronic renal failure, chronic liver failure, central nervous system tumors, multiple sclerosis, Parkinson's disease, or radical pelvic surgeries.

As used herein, "administer" or "administration" or "injection" or "provide" refers to a technique used to deliver a substance, i.e., stem cells or mesenchymal stem cells, into the body systemically or locally, or any combination thereof. When administering a therapeutically effective amount of the present invention parenterally or intravenously, it is generally formulated in a unit dosage injectable form (e.g., solution, suspension, or emulsion). The pharmaceutical formulations suitable for injection may include sterile aqueous solutions or dispersions and sterile powders for reconstitution into sterile injectable solutions or dispersions.

As used herein, a "therapeutically effective amount" that can be administered to a male, a transgendered male, or a male-like subject (e.g., the cavernous nerve of a penis of a male subject) to treat an erectile dysfunction (e.g., promote an erection) within less than 60 minutes, less than 45 minutes, less than 30 minutes, or less than 15 or less than 5 minutes of application.

As used herein, the term "therapeutically effect amount" also means the amount of amniotic fluid stem cells or human amniotic fluid stem cells, that when administered to an individual for treating a state, disease, disorder or condition associated with or caused by cavernous nerve injury is sufficient to effect such treatment. The therapeutically effective amount will vary depending on the particular state, disease, disorder or condition being treated and its severity and the age, weight, physical condition and responsiveness of the subject to be treated. Thus one or more of these parameters can be used to select and adjust the therapeutically effective amount of amniotic fluid stem cells or human amniotic fluid stem cells. The therapeutically effective amount or therapeutically effect amount of hAFSC ranges from 1*10⁵ and up, such as 1*10⁶, 1*10⁷, 1*10⁸, 1*10⁹, 2*10¹, 2*10², 2*10³, 2*10⁴, 2*10⁵, 1*10⁶, 2*10⁷, 2*10⁸ or more.

As used herein, the term "secretome of paracrine" or "secretome" or "stem-cell derived secretome" or "stem-cell derived secretome of paracrine" or "mesenchymal stem cell-derived secretomes" or "hAFSCs secretome" are used interchangeably. In certain aspects, secretomes contain lipids, proteins, RNAs, and microRNAs and MSC-secreted secretomes are involved in cardioprotective paracrine effects. In some embodiments of the present application, MSC-derived secretomes may increase angiogenesis, viability, or proliferation. Further, the secretomes may protect against vascular injuries and repair such as cardiac injury, prostate injury, liver injury, or other organs of injury.

As used herein "vWF," "vWF factor," or "von Willebrand factor" is a clinical marker of risk associated with atherosclerosis or a marker of endothelial cells.

It has been found that the implantation of mesenchymal stem cells (MSCs) into the cavernous nerve attached to the corpora cavernosum, migrate into the target area, where the MSCs differentiate into endothelial cells and smooth muscle cells and then proliferate and form structures including myocardium, coronary arteries, arterioles, and capillaries, restoring the erectile function in various subjects.

In at least one embodiment, the present invention extends to a stem cell or amniotic fluid stem cell, derived from non-embryonic animal cells or tissue, capable of self-regeneration and capable of differentiation to cells of endodermal, ectodermal and mesodermal lineages, but not limited thereto.

In a particular aspect, the present invention extends to a mesenchymal stem cells, derived from prenatal animal cells or tissue or amniotic fluid, capable of self-regeneration and capable of differentiation to cells of endodermal, ectodermal and mesodermal lineages, but not limited thereto.

As used herein, "corpus cavernosum" refers to one of the main tissue components of the penile erectile tissue, which may include smooth muscle and endothelial cells, but not limited thereto.

As used herein "cavernous nerve" refers to the nerve that facilitates penile erection, which the cavernous nerve arises from pelvic splanchnic nerves plexus to prostate plexus. The cavernous nerve contains both sympathetic and parasympathetic fibers derived from pelvic plexus; the cavernous nerve leaves the pelvis between the transverse perineal muscles and membranous urethra before passing beneath the pubic arch to supply each corpus cavernosum and cavernous nerve supplies the corpus cavernosum and penile urethra, and terminates in a delicate network around the erectile tissue, but not limited thereto.

As used herein, "amniocentesis" may be a procedure utilize to obtain 1 to 40 mL, e.g., 2 to 5 mL, 5 to 10 mL, 10 to 20 mL, 20 to 30 mL, or 30 to 40 mL of amniotic fluid from a female subject by inserting a long spinal needle, having a sharp-cutting tip, through the surface of the skin and into the uterine cavity and obtaining the amniotic fluid by aspiration.

As used herein, "medium," "basal medium," or "media" refers to an optimal culture medium for the cultivation of a variety of animal cells, including neurons, stem cells, mesenchymal stem cells, primary epithelial cells, keratinocytes, cervical epithelial cells, kidney epithelial cells, and established cell lines, but not limited thereto. In at least one embodiment, the cultivation may be expansion or differentiation.

It is further noted that, as used in this disclosure, "cavernous nerve injury" is used interchangeably with the term "cavernous nerve crush," which may include transection, excision, freezing, crushing, unless the context clearly indicates otherwise, but not limited thereto. In an aspect of penile smooth muscle contraction, particularly the molecular mechanism of penile smooth muscle contraction, the penile smooth muscle contraction and relaxation is regulated by cytosolic free Ca²⁺. Norepinephrine from nerve endings and endothelins and prostaglandin F2α from endothelium activate receptors on smooth muscle cells to increase inositol triphosphate and diacylglycerol resulting in release of calcium from intracellular stores such as sarcoplasmic reticulum and/or opening of calcium channels on the smooth muscle cell membrane leading to an influx of calcium from extracellular space. This triggers a transient increase in cytosolic free Ca²⁺ from a resting level of for example, 120 to 270 to 500 to 700 nM. Further, at the elevated level, Ca²⁺ binds to calmodulin and changes the latter's conformation to expose sites of interaction with myosin light-chain kinase. The resultant activation catalyzes phosphorylation of myosin light chains and triggers cycling of myosin crossbridges (heads) along actin filaments and the development of force. In addition, phosphorylation of the light chain also activates myosin ATPase, which hydrolyzes ATP to provide energy for muscle contraction. (Dean RC et al., Urol Clin North Am, 32(4):379, 2005).

As used therein, "nerve-sparing prostatectomy" involves dissecting the nerve bundle off of the side of the prostate.

As used herein, "sham" or "sham surgery" refers to the control group, or the placebo.

The MSCs of the present invention may be isolated from the non-embryonic tissue selected from the group of muscle, dermis, fat, tendon, ligament, perichondrium, periosteum, heart, aorta, endocardium, myocardium, epicardium, large arteries and veins, granulation tissue, peripheral nerves, peripheral ganglia, spinal cord, dura, leptomeninges, trachea, esophagus, stomach, small intestine, large intestine, liver, spleen, pancreas, parietal peritoneum, visceral peritoneum, parietal pleura, visceral pleura, urinary bladder, corpus cavernosum, corpus spongiosum, urethra, gall bladder, kidney, placental tissues, acellular amnion, amnionic fluid, associated connective tissues or bone marrow.

In at least one embodiment of the present disclosure, ED may be caused by a number of chronic illness and factors, including vascular disease, stroke, diabetes, hormonal insufficiencies (for example, hypogonadism), neurological disorders (for example, Parkinson's disease and trauma from radical prostatectomy), multiple sclerosis, radical pelvic surgeries, psychological state and trauma including brain and spinal injury, but the present disclosure is not limited thereto. In some embodiments of the present disclosure, neurogenic ED is the common complication after radical prostatectomy of prostate cancer. In some embodiments of the present disclosure, the subject is suffered from ED as a side-effect of the administration of certain medications such as diuretics, anti-hypertensives, anti-histamines, anti-depressants, Parkinson's disease drugs, anti-arrhythmic, tranquilizers, muscle relaxants, non-steroid anti-inflammatory drugs, histamine H2-receptor antagonists, hormones, chemotherapy medications, prostate cancer drugs, anti-seizure medications, but the present disclosure is not limited thereto.

In at least one embodiment of the present disclosure, a high intracavernous pressure (ICP) is maintained with a low inflow rate.

In at least one embodiment of the present disclosure, ED may be improved by increasing blood flow to the penis via enhance the effect of nitric oxide which causes an increase in blood blow.

In at least one embodiment of the present disclosure, the underlying mechanisms of ED may be vasculogenic, neurogenic, anatomical, hormonal, drug-induced and/or psychogenic.

In at least one embodiment of the present disclosure, the MSCs of the present invention may be isolated from non-human cells or human cells or amniotic cells or ammonitic fluid of human, but not limited thereto.

These MSCs, complexes or secretomes may be used for a variety of purposes, such as treatment or prevention for various organs or organ system failure, for example, heart failure, liver failure, or erectile dysfunction, but not limited thereto.

In at least one embodiment of the present disclosure, a method of treatment of erectile dysfunction may include providing, administering, or giving animal amniotic fluid stem cells, human amniotic fluid stem cells, mesenchymal stem cells, cord blood stem cells, placental stem cells, bone marrow stem cells, adipose tissue derived stem cells, and any other type of stem cells.

In at least one embodiment of the present disclosure, changes of penile tissue after CN injury may include a decrease in intracorporal pressure, apoptosis of smooth muscle and endothelium cell, a reduce in neural-Nitric oxide synthase (nNOS) nerve fiber density, an up-regulated fibroproliferative cytokines (e.g., TGFβ1), or abnormal biological signaling responses (e.g., ROS). In some embodiments, it is possible to use MSCs and to treat or prevent the onset of erectile dysfunction by providing an amount of mesenchymal stem cells of the amniotic fluid is administered from about 1*10⁶, 1*10⁷, 1*10⁸, 1*10⁹, 2*10¹, 2*10², 2*10³, 2*10⁴, 2*10⁵, 2*10⁶, 1*10⁶, 2*10⁷, or 2*10⁸. In some embodiments, the dosage forms of the hAFSC in saline are 4x10⁵ cells with 2x10⁵ cells on each side, 2x10⁶ cells with 1x10⁶ cells on each side, or 1x10⁷ cells with 5x10⁶ cells on each side. Some embodiments of the present disclosure provide an administering method to the subject a therapeutically effective amount of a plurality of mesenchymal stem cells, wherein the plurality of MSCs is obtained from at least an amniotic fluid. These MSCs can be used to improve smooth muscle relation, an arterial dilation, a venous restriction and neuronal atrophy, but not limited thereto.

Tissues of the corpus cavernosum were collected and the histological analysis was performed. Various analytical methods may include, for example, Hematoxylin-eosin staining, H&E staining and Masson's trichrome staining, collagen staining and Roxarco Luxol fast blue staining, Western blotting method and immunofluorescence staining analysis to verify the specific protein expression (α-SMA, eNOS, nNOS, iNOS, beta-III tublin, NF-1, and von Willebrand Factor), Terminal deoxyribonucleotidyl transferase-mediated nick end labeling analysis (TUNEL assay), reverse transcription and quantitative real-time polymerase chain reaction (PCR) to evaluate gene expression of fibrosis.

### EXAMPLE

Exemplary embodiments of the present disclosure are further described in the following examples, which should not be construed to limit the scope of the present disclosure.

### Example 1: Animal Model and Experimental Design

Twelve-weeks-old Sprague-Dawley male rats were obtained from BioLasco, Taiwan Co., Ltd. (Taipei, Taiwan). All rats were housed under standard laboratory conditions, and the animal study was reviewed and approved by the Fu Jen Catholic University Animal Care and Use Committee.

For the surgical procedure, rats were anesthetized using an intraperitoneal injection of sodium pentobarbital (40 mg/kg). A lower midline abdominal incision was made after the abdomen was shaved and prepared with an iodine-based solution. Subsequently, the prostate gland was exposed, and the bilateral posterolateral CNs and major pelvic ganglion (MPGs) were identified. There was no further surgical manipulation in the sham condition. In BCNC group, the CNs were isolated, a crush injury was applied using a hemostat clamp (Roboz Surgical Instrument Co., Inc., Gaithersburg, MD) for 2 minutes, and the abdomen was closed using 2 layers of suturing. This procedure resulted in a BCNC injury. In Nerve-sparing prostatectomy (CNSP) group, a minimal cutting of the nerve which involves the intrafascial and subextrafascial dissection.

The preparation of human amniotic fluid mesenchymal stem cell (hAFMSC), as an example, was based on a method of harvesting mesenchymal stem cells from human amniotic fluid uses a two-stage culture protocol comprising culturing human amniocytes and then culturing mesenchymal stem cells. For culturing human amniocytes, as an example, using primary amniocyte cultures according to a routine or standard amniocytes culture protocol that was conducted in a cytogenetic laboratory (US7101710B2).

The method for harvesting mesenchymal stem cells from human amniotic fluid comprising:
(a) culturing a plurality of human amniotic fluid amniocytes including the steps of: (i) setting up primary amniocyte cultures comprising a plurality of adherent human amniotic fluid cells and a supernatant containing a plurality of non-adherent human amniotic fluid cells and a liquid medium; and (ii) collecting said non-adherent human amniotic fluid cells in the supernatant; and
(b) culturing a plurality of mesenchymal stem cells including the steps of: (i) centrifuging the non-adherent human amniotic fluid cells; (ii) plating the centrifuged non-adherent human amniotic fluid cells from step b(i) in an α-modified Minimum Essential Medium supplemented with fetal bovine serum in a culture flask; and (iii) incubating said plated non-adherent human amniotic fluid cells from step (b)(ii) with humidified CO2 to promote mesenchymal stem cell growth.

The method for harvesting secretome from human amniotic fluid comprising: wherein the secretome is prepared by the method of following steps: culturing amniotic fluid stem cells in a basal medium for 24 to 72 hours when the cells reached 80% confluence; collecting a supernatant of the culture medium after centrifuging at 300 xg for 10 min to eliminate, non-permanently damaged cells, permanently damaged cells, dead cells and debris. Then, the supernatant was filtered using a 0.22 µm filter and stored at -20 °C.

### Example 2: Treatment of ED due to BCNC by Administration of Human Amniotic Fluid Stem Cells

The experimental design and surgical procedures were generated as described in Example 1. Specifically, the rats were randomly assigned to three groups: sham group (n=8), bilateral cavernous nerve crush (BCNC) injury group (n=8) and BCNC-treated with hAFSC group (n=8). After harvesting the mesenchymal stem cells from human amniotic fluid, about 1x10⁶ cells in 200 µL hAFSC were administered via the injury site. As shown in FIG. 1A-1, the injection according to the invention, e.g., the intracavernous injection of the hAFSC, provides a recovery of ICP vs time curve, suggesting spontaneous neuroregeneration. The maximum ICP of the hAFSC group were significantly higher than the maximum ICP of the BCNC group. As shown in FIGS. 1A-2 to 1A-5, the hAFSC group in all figures were significantly higher than the BCNC group except 1A-4, mean arterial pressure (MAP) which remained the normal region. Accordingly, aspects of the invention provide methods for delivering an effective treatment to the subject to treat or prevent erectile dysfunction.

### Example 3: Treatment of ED due to BCNC by Administration of hAFSC secretome

The experimental design and surgical procedures are generated as described in Example 1, but administrating of hAFSCs secretome. Specifically, the rats were randomly assigned to three groups: sham group (n=8), BCNC group (n=8), and BCNC injury treated with hAFSCs secretome group (n=8). After BCNC injury, 200 µL of hAFSC secretome were administered via the injury site each week for 4 times. As shown in FIG. 1B-5, the maximum ICP/MAP of the BCNC injury treated with hAFSCs secretome treatment group according to the present invention showed a higher ratio than the ratio of the BCNC group. Accordingly, aspects of the invention provide methods for delivering an effective treatment, delivering hAFSCs secretome to the injury site to the subject to treat or prevent erectile dysfunction.

### Example 4: Treatment of ED due to CNSP by Administration of Human Amniotic Fluid Stem Cells

The experimental design and surgical procedures are generated as described in Example 1. The rats were randomly assigned to three groups: sham group (n=8), CNSP group (n=8) and CNSP-treated with hAFSC group (n=8). After harvesting the mesenchymal stem cells from human amniotic fluid, about 1x10⁶ cells in 200 µL hAFSC were administered via the injury site due to CNSP. As shown in FIG. 1C-1, the injection according to the invention, e.g., the intracavernous injection of the hAFSC, provides a recovery of ICP vs time curve, in which the maximum ICP of the hAFSC group were significantly higher than the maximum ICP of the CNSP group. As shown in FIG. 1C-5, the maximum ICP/MAP of the hAFSC group according to the present invention showed a significantly higher ratio than the ratio of the CNSP group. Accordingly, aspects of the invention provide methods for delivering an effective treatment to the subject to treat or prevent erectile dysfunction.

### Example 5: Treatment of ED due to CNSP by Administration of hAFSC Secretome

The rats were randomly assigned to three groups: sham group (n=8), CNSP group (n=8) and CNSP-treated hAFSC secretome group (n=8). After CNSP injury, 200 µL of hAFSC secretome were administered via the injury site each week for 4 times. As shown in FIG. 1D-1, the injection according to the invention, e.g., the intracavernous injection of the hAFSC secretome, provides a recovery of ICP vs time curve, in which the maximum ICP of the secretome group were significantly higher than the maximum ICP of the CNSP group. As shown in FIGS. 1D-2 to 1D-5, the maximum ICP/MAP of the CNSP group treated with hAFSC secretome showed a significantly higher ratio than the ratio of the CNSP group.

In addition, as shown in FIG. 1E-1, the ED of the rats due to BCNC treated with hAFSC or Secretome group exhibited a significantly higher maximum ICP, about 100 cmH₂O pressure relative to the BCNC group. As shown in FIG. 1E-6, the delta ICP to MAP ratio of the ED due to BCNC treated with hAFSC or Secretome group was more than 0.5, a significantly higher ratio relative to the BCNC group. Accordingly, aspects of the invention provide methods for delivering an effective treatment to the subject to treat or prevent erectile dysfunction.

Similarly, as shown in FIG. 1F-1, the ED due to CNSP treated with hAFSC or Secretome group exhibits a significantly higher maximum ICP, about 100 cmH₂O pressure relative to the CNSP group. As shown in FIG. 1F-6, the delta ICP to MAP ratio of the ED due to CNSP treated with hAFSC or Secretome group was more than 0.5, a significantly higher ratio relative to the CNSP group that was less than 0.2.

### Example 6: Immunofluorescence staining for the Dorsal Penile Nerve

Tissues of the corpus cavernosum were observed under Transmission Electron Microscope for the ultra-fine tissue changes, such as changes in mitochondrial shape. The effectiveness of amniotic fluid stem cells in treating cavernous nerve injury in rats and the preliminary effective mechanism were confirmed after statistical analysis.

Immunofluorescence staining of nNOS and β-III tubulin were demonstrated and expressed in the nerve fibers of the dorsal penile nerve of sham, BCNC and hAFSC groups, as shown in FIG. 2A and FIG. 2B. The nNOS expression in dorsal penile nerve is significant lower in BCNC group comparing to both the sham and hAFSC group (*p* < 0.05). The nNOS-positive nerve fibers of the dorsal penile nerve were immunostained for β-III-tubulin to identify nerve fibers positive for nNOS and to quantify their nNOS, as shown in the immunofluorescence staining in FIGS. 3A-1. As shown in FIG. 3A-4, the ratio of the area of nNOS/β-III-tubulin expression was significantly lower in the BCNC group relative to the sham and hAFSC groups (*p* < 0.05). Ultrastructural analysis of the cavernous nerve in FIG. 7A also demonstrated the smaller circular nerve fiber and myelin debris in the BCNC group, visually compared to the sham and hAFSC group. Similarly, ultrastructural analysis of the cavernous nerve in FIG. 7B also demonstrated the smaller circular nerve fiber and myelin debris in the CNSP group, visually compared to the sham and hAFSC group. Similarly, ultrastructural analysis of the cavernous nerve in FIG. 7C also demonstrated the smaller circular nerve fiber and myelin debris in the CNSP group, visually compared to the sham and Secretome group. With the treatment of Secretome or hAFSC, the amount of myelin debris decreased and myelin began to regenerate and a thicker myelin sheath was observed.

### Example 7: Immunofluorescence staining for the Corpus Cavernosum

The immunofluorescence expression of vWF as shown in FIG. 5A-1 and α-SMA as shown in FIG. 6A-1, FIG. 6A-2 were also presented in sham, BCNC, and hAFSC group. The vWF expression of BCNC and hAFSC group was significantly lower than the vWF expression of the Sham group (*p* < 0.05), while BCNC group was also significantly different from hAFSC group (*p* < 0.05), while BCNC group was also significantly different from the hAFSC group (*p* < 0.05). The immunofluorescence expression of α-SMA in the other hand, only show a significant difference in BCNC group compared to both Sham and hAFSC groups. The integrity of corpus cavernosum tissue were also showed in ultrastructural analysis of the cavernous nerve (FIG. 7A), demonstrating a thicker and tighter muscle layers in Sham and hAFSC group, comparing to the BCNC group.

### Statistical analysis

Data were expressed as mean± standard deviation. Differences between the means of multiple treatment groups were tested by ANOVA and the Scheffe *post hoc* test, with a statistical significance determined at *P* < 0.05. Statistical analysis was performed using SPSS v.12.0 (SPSS Inc., Chicago, IL) for Windows.

The above-described descriptions of the detailed embodiments are to illustrate the preferred implementation according to the present disclosure, and it is not to limit the scope of the present disclosure. Accordingly, all modifications and variations completed by those with ordinary skill in the art should fall within the scope of the present disclosure defined by the appended claims.

### Example 8: Therapeutic effect amount of human amniotic fluid stem cells on bilateral cavernous nerve injury

The experimental design and surgical procedures are generated as described in Example 1, but administrating various concentrations of hAFSC, for example, 2*10⁴ hAFSC, 2*10⁵ hAFSC, 2*10⁶ hAFSC, 1*10⁷ hAFSC, and 2*10⁷ hAFSC. Specifically, the rats were randomly assigned to seven groups: sham group (n=8), bilateral cavernous nerve crush (BCNC) injury group (n=7), and BCNC-treated with hAFSC group in an amount of 2*10⁴ (n=5), 2*10⁵ (n=5), 2*10⁶ (n=14), 1*10⁷ (n=6), and 2*10⁷ (n=7). After harvesting the mesenchymal stem cells from human amniotic fluid, about 2*10⁴, 2*10⁵, 2*10⁶, 1*10⁷, and 2*10⁷cells in 200 µL hAFSC were administered via the injury site to each group. As shown in FIG. 8A-7, the injection according to the invention, e.g., the intracavernous injection of the hAFSC, provides a recovery of ICP vs time curve, suggesting spontaneous neuroregeneration and the recovery effect is most significant in the 2*10⁵ hAFSC group. In other words, the therapeutic effect amount of hAFSC to treat a nerve injury, for example, bilateral cavernous nerve crushing, is most effective at a cell amount of 2*10⁵. The maximum ICP of the hAFSC groups of all concentrations tested were significantly higher than the maximum ICP of the BCNC group. As shown in FIGS. 8A-1 to 8A-6, the hAFSC groups were significantly higher than the BCNC group except 8A-4, mean arterial pressure (MAP) in the 2*10⁴ hAFSC group which was lower than the BCNC group. FIG. 10 shows that the smooth muscle to collagen ratio has improved significantly in the 2*10⁵ hAFSC group, which is consistent and reflected in the H&E and Masson staining of FIG. 9. Accordingly, aspects of the invention provide the therapeutic effect amount for delivering an effective treatment to the subject to treat or prevent erectile dysfunction. In FIG. 11, nerve fibers of the dorsal penile nerve were immunostained for β-III tublin to identify fibers positive for nNOS and quantify their nNOS content. The quantitative analyses in FIG. 12 indicated that the number of nNOS-positive nerve fibers was dramatically reduced in the BCNC groups compared with the sham group; however, there was a significant increase in the number of nNOS-positive nerve fibers in all hAFSC groups in various concentrations, compared with the BCNC group. The increase in the number of nNOS-positive nerve fibers is also most significantly in the 2*10⁵ and 2*10⁶ hAFSC groups, which is consistent and reflected in the immunofluorescence staining in FIG. 11 for nNOS, β-III tublin, and NF-1 in the dorsal penile nerve.

Furthermore, the smooth muscle cell content in the corpora cavernous was evaluated by α-smooth muscle actin staining in which the smooth muscle cell content in the corpora cavernous was significantly lesser in the BCNC group compared with the sham and hAFSC treated groups. As shown in FIG. 14, the increase in the number of α-smooth muscle actin is also most significantly in the 2*10⁵ and 2*10⁶ hAFSC groups, which is consistent and reflected in the immunofluorescence staining images in FIG. 13. Similarly, as shown in FIG. 15, the increase in the number of vwf expression is also most significantly in the 2*10⁵ and 2*10⁶ hAFSC groups, which is consistent and reflected in the immunofluorescence staining images in FIG. 13.

## Claims

1. Mesenchymal stem cells derived from amniotic fluid for use in treating erectile dysfunction, **characterized by** comprising administering to a subject in need thereof with a therapeutically effective amount of the mesenchymal stem cells derived from amniotic fluid and harvested by a two-stage culture protocol.

2. The mesenchymal stem cells derived from amniotic fluid for use of claim 1, wherein the erectile dysfunction is a neurogenic erectile dysfunction caused by stroke, brain and/or spinal injuries, diabetes, multiple sclerosis, Parkinson's disease, trauma from radical prostatectomy or radical pelvic surgeries, or any combination thereof.

3. The mesenchymal stem cells derived from amniotic fluid for use of claim 1, wherein the mesenchymal stem cells are administered to a subject in need thereof in a therapeutically effective amount of 2*10⁵, 1*10⁶, or 2*10⁶ cells.

4. The mesenchymal stem cells derived from amniotic fluid for use of claim 1, wherein the mesenchymal stem cells are positive for CD 73, CD 90, CD 105, Nestin, Sox2, or any combination thereof; the mesenchymal stem cells are negative for CD 34, CD 45, CD 14, CD 11b, CD 79α, CD 19, HLA-DR, or any combination thereof; and the mesenchymal stem cells have a fibroblast-like morphology in an attachment culture.

5. The mesenchymal stem cells derived from amniotic fluid for use of claim 1, wherein the mesenchymal stem cells have an osteogenic differentiability, an adipogenic differentiability, a chondrogenic differentiability, or any combination thereof.

6. The mesenchymal stem cells derived from amniotic fluid for use of claim 1, wherein the mesenchymal stem cells from human amniotic fluid is obtained from method comprising:
(a) culturing a plurality of human amniotic fluid amniocytes including the steps of:
(i) setting up primary amniocyte cultures comprising a plurality of adherent human amniotic fluid cells and a supernatant containing a plurality of non-adherent human amniotic fluid cells and a liquid medium; and
(ii) collecting said non-adherent human amniotic fluid cells in the supernatant; and
(b) culturing a plurality of mesenchymal stem cells including the steps of:
(i) centrifuging the non-adherent human amniotic fluid cells;
(ii) plating the centrifuged non-adherent human amniotic fluid cells from step b(i) in an alpha-modified Minimum Essential Medium supplemented with fetal bovine serum in a culture flask; and
(iii) incubating said plated non-adherent human amniotic fluid cells from step (b)(ii) with humidified CO2 to promote mesenchymal stem cell growth.

7. The mesenchymal stem cells derived from amniotic fluid for use of claim 6, wherein the α-modified minimum essential medium is further supplemented with about 4 ng/ml basic fibroblast growth factor.

8. A secretome derived from mesenchymal stem cells for use in treating erectile dysfunction, **characterized by** comprising administering to a subject in need thereof with a therapeutically effective amount of secretome derived from mesenchymal stem cells, wherein the mesenchymal stem cells are derived from amniotic fluid, and the mesenchymal stem cells are harvested by a two-stage culture protocol.

9. The secretome derived from mesenchymal stem cells for use of claim 8, and the secretome derived from mesenchymal stem cells is administered to a subject in need thereof with a therapeutically effective amount each week for 4 times.

10. The secretome derived from mesenchymal stem cells for use of claim 8, wherein the erectile dysfunction is neurogenic erectile dysfunction caused by stroke, brain and/or spinal injuries, diabetes, multiple sclerosis, Parkinson's disease, trauma from radical prostatectomy or radical pelvic surgeries, or any combination thereof.

11. The secretome derived from mesenchymal stem cells for use of claim 8, wherein the mesenchymal stem cells are positive for CD 73, CD 90, CD 105, Nestin, Sox2, or any combination thereof, and mesenchymal stem cells are negative for CD 34, CD 45, CD 14, CD 11b, CD 79α, CD 19, HLA-DR, or any combination thereof.

12. The secretome derived from mesenchymal stem cells for use of claim 8, wherein the mesenchymal stem cells from human amniotic fluid is obtained from method comprising:
(a) culturing a plurality of human amniotic fluid amniocytes including the steps of:
(i) setting up primary amniocyte cultures comprising a plurality of adherent human amniotic fluid cells and a supernatant containing a plurality of non-adherent human amniotic fluid cells and a liquid medium; and
(ii) collecting said non-adherent human amniotic fluid cells in the supernatant; and
(b) culturing a plurality of mesenchymal stem cells including the steps of:
(i) centrifuging the non-adherent human amniotic fluid cells;
(ii) plating the centrifuged non-adherent human amniotic fluid cells from step b(i) in an alpha-modified Minimum Essential Medium supplemented with fetal bovine serum in a culture flask; and
(iii) incubating said plated non-adherent human amniotic fluid cells from step (b)(ii) with humidified CO2 to promote mesenchymal stem cell growth.

13. The secretome derived from mesenchymal stem cells for use of claim 12, wherein the mesenchymal stem cells have a fibroblast-like morphology in an attachment culture.

14. The secretome derived from mesenchymal stem cells for use of claim 8, wherein the secretome of mesenchymal stem cells is obtained by steps comprising:
(a) culturing amniotic fluid stem cells in a basal medium for about 24 to 72 hours when the cells reached about 80% confluence;
(b) collecting a supernatant of the culture medium after centrifuging at about 300 xg for about 10 minutes to eliminate dead cells and debris; and
(c) filtering the supernatant with a 0.22-µm filter and storing the secretome at about -20 °C.
